# EUROPÄISCHE PATENTSCHRIFT

(11) **EP 1 972 307 B1**
(45) Veröffentlichungstag und Bekanntmachung des Hinweises auf die Patenterteilung: **27.10.2010**
(21) Anmeldenummer: 08001687.6
(22) Anmeldetag: 30.01.2008
(51) Int. Cl.: A61F 2/18

(54) **Gehörknöchelchenprothese mit sensibler Kopfplatte**
Auditory ossicle prosthesis with sensitive head plate.
Prothèse d'osselets de l'ouïe dotée d'une plaque frontale sensible.

(30) Priorität: 22.03.2007 DE 102007013708
(43) Veröffentlichungstag der Anmeldung: 24.09.2008
(73) Patentinhaber: Heinz Kurz GmbH Medizintechnik, 72144 Dusslingen (DE)
(72) Erfinder: Hüttenbrink, K.-B., Prof.Dr., 50931 Köln (DE); Steinhardt, Uwe, 72145 Hirrlingen (DE)
(74) Vertreter: Kohler Schmid Möbus

(56) Entgegenhaltungen:
- EP-A- 1 181 907
- DE-A1- 19 647 579
- DE-U1- 20 300 723
- DE-U1- 29 819 892
- DE-U1-202005 003 782
- US-A1- 2006 058 875

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Verbindung mit dem Trommelfell ausgebildetes erstes Befestigungselement und an ihrem anderen Ende ein zweites Befestigungselement zur mechanischen Verbindung mit einem Glied oder Teilen eines Gliedes der Gehörknöchelchenkette oder mit dem Innenohr sowie ein die beiden Befestigungselemente Schall leitend miteinander verbindendes Verbindungselement umfasst, und wobei die Kopfplatte einen radial äußeren Ringbereich, einen radial inneren, insbesondere zentralen Ankoppelbereich zum mechanischen Ankoppeln der Kopfplatte an das Verbindungselement sowie mehrere Stegelemente zur radialen Verbindung des radial äußeren Ringbereichs mit dem zentralen Ankoppelbereich aufweist.

Eine derartige Vorrichtung ist bekannt aus der DE 20 2005 003 782 U1.

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einher gehende Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sog. Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet uns meistens ais iängiicher Schaft ausgeführt ist. Eine solche in sich starre, aber gegenüber dem Verbindungselement verkippbare Kopfplatte ist u.a. beschrieben in der US 2004/0162614 A1, in dem Artikel M.W. YUNG, Ph.D., F.R.C.S., D.L.O., C. BREWIS, F.R.C.S., "A comparison of the user-friendliness of hydroxyapatite and titanium ossicular prostheses", The Journal of Laryngology & Otology, February 2002, Vol. 116, pp. 97-102, oder beispielsweise auch in der US 2006/0271190 A1.

Nachteilig bei diesen bekannten Gehörknöchelchenprothesen ist allerdings, dass durch die starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Aufgabe der vorliegenden Erfindung ist es demgegenüber, eine gattungsgemäße Vorrichtung der eingangs beschriebenen Art mit möglichst einfachen technischen Mitteln dahin gehend zu verbessern, dass unaufwändig und kostengünstig eine hohe postoperative Flexibilität und Variabilität der Prothese erreicht wird, wobei die Qualität der Schallleitung durch die Prothese erheblich erhöht werden soll, ohne dass es zu den oben geschilderten Komplikationen kommen kann.

Erfindungsgemäß wird diese Aufgabe auf ebenso überraschend einfache wie wirkungsvolle Art und Weise dadurch gelöst, dass die Kopfplatte eine Dicke t zwischen 0,01mm und 0,25mm sowie einen minimalen Durchmesser D zwischen 2mm und 5mm und die Stegelemente eine maximale Breite b zwischen 0,01mm und 0,2mm aufweisen, so dass die Stegelemente bei lokaler Medialbewegung des Trommelfells dieser Medialbewegung lokal folgen, jedoch die Bewegung nicht an entfernte Bereiche der Kopfplatte weitergeben. Durch diese flexible Gestaltung der Gehörknöchelchenprothese wird bei einer solchen kleineren Medialbewegung des Trommelfells eine starre Verkippung der gesamten Kopfplatte vermieden. Vielmehr verwindet sich die Kopfplatte lokal in sich, wobei sie jedoch bei großflächigen, durch Schall verursachten Bewegungen des Trommelfells diese an das Verbindungselement weitergibt, so dass eine optimale Übertragung des Schalls vom Trommelfell zum Mittelohrraum hin gewährleistet wird.

Dadurch werden auf simple Weise die Vorteile der oben beschriebenen bekannten Gehörknöchelchenprothese gemäß der gattungsbildenden DE 20 2005 003 782 U1 genutzt, wobei aber auch die Vorteile der in den oben genannten US 2004/0162614 A1 und US 2006/0271190 A1 beschriebenen Prothesen erhalten bleiben und die gemeinsamen Nachteile einer starren Verkippung der Kopfplatte vermieden werden.

Eine besonders bevorzugte Ausführungsform der erfindungsgemäßen Gehörknöchelchenprothese zeichnet sich dadurch aus, dass die Stegelemente eine maximale Breite b und der radial äußere Ringbereich eine maximale Breite B aufweisen, und dass gilt: 2b < B.

Um die gewünschte Flexibilität zu erreichen, sollte bei einer maximalen Breite b der Stegelemente und einem minimalen Durchmesser D der Kopfplatte einschließlich des Ringbereiches gelten: b ≤ 0,05D, vorzugsweise b ≈ 0,03D. Beim Stand der Technik, etwa bei den in der US 2004/0162614 A1 beschriebenen Gehörknöchelchenprothesen, liegt das Verhältnis b/D mindestens bei 0,1 oder darüber.

Besonders bevorzugt sind Ausführungsformen der Erfindung, bei denen die Stegelemente in der Kopfplattenebene nicht geradlinig, sondern auf mehrfach gekrümmten Kurven verlaufen, wodurch sich die erwünschte Wirkung einer lokalen Flexibilität der Kopfplatte und ein lediglich lokal begrenztes Ausweichen bei kleineren Medialbewegungen des Trommelfells noch verstärkt. Außerdem kann dadurch die Kopfplatte einer eventuellen post-operativen Veränderung des Trommelfells leichter folgen.

Bei weiteren vorteilhaften Ausführungsformen der erfindungsgemäßen Gehörknöchelchenprothese ist jedes Stegelement mit mindestens zwei anderen Stegelementen verbunden, so dass eine Art Netzwerk aus Stegelementen entsteht.

Um die gewünschte Flexibilität zu erhöhen, können bei einer Klasse von Ausführungsformen die Stegelemente zwischen dem zentralen Ankoppelbereich und dem radial äußeren Ringbereich ununterbrochen verlaufen.

Alternativ können aber auch zumindest einige der Stegelemente eine Unterbrechung zwischen dem zentralen Ankoppelbereich und dem radial äußeren Ringbereich aufweisen.

Ebenso kann bei einer weiteren Klasse von Ausführungsformen der Erfindung der radial äußere Ringbereich ununterbrochen in sich selbst geschlossen verlaufen. Dadurch kann bei einer Bildung von post-operativen Retraktionen die Ausbildung von den das Trommelfell gerichteten Spitzen vermieden werden.

Bei alternativen Ausführungsformen der erfindungsgemäßen Gehörknöchelchenprothese hingegen weist der radial äußere Ringbereich mindestens eine, vorzugsweise mehrere Unterbrechungen auf.

Wie aus dem Stand der Technik an sich bekannt, kann der radial äußere Ringbereich eine ovale oder kreisrunde Form aufweisen.

Vorteilhaft können aber auch alternative Ausführungsformen der Erfindung sein, bei denen der radial äußere Ringbereich eine Schlangenlinienförmige Außenkontur aufweist.

Zur Erleichterung des operativen Einsetzens der erfindungsgemäßen Gehörknöchelchenprothese können sich weitere Ausführungsformen geometrisch dadurch auszeichnen, dass der radial äußere Ringbereich eine einseitige Einbuchtung zur Aufnahme des Hammergriffs aufweist.

In der Regel wird bei der erfindungsgemäßen GehörknöchelchenProthese die Kopfplatte am einen Ende eines länglichen Schafts angeordnet sein, der die Kopfplatte mit dem anderen Ende der Gehörknöchelchenprothese verbindet, wie dies an sich aus dem Stand der Technik wohlbekannt ist.

Um die oben erörterte Flexibilität bzw. Variabilität der Prothese zu erreichen, wie sie an sich in der EP 1 181 907 B1 beschrieben ist, kann bei einer besonders bevorzugten Ausführungsform am oder im länglichen Schaft ist mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Alternativ kann der Schaft bei besonders einfachen und preisgünstig herstellbaren Ausführungsformen der erfindungsgemäßen Prothese aber auch einstückig durchgehend, insbesondere starr ausgeführt sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In sämtlichen Ausführungsformen der Erfindung wird das erste Befestigungselement eine zur Anlage am Trommelfell ausgebildete Kopfplatte umfassen. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über die Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbundwerkstoffen hergestellt sind. Mit diesen Materialien können postoperative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine schematische räumliche Darstellung einer Ausführungsform der erfindungsgemäßen Gehörknöchelchenprothese mit einem Stempel als zweiten Befestigungselement am anderen Ende des länglichen Schaftes in einer Draufsicht auf die Kopfplatte; und
- Fig. 2: eine Ausführungsform mit einem glockenförmigen zweiten Befestigungselement in einer Ansicht von unter her auf die Glocke.

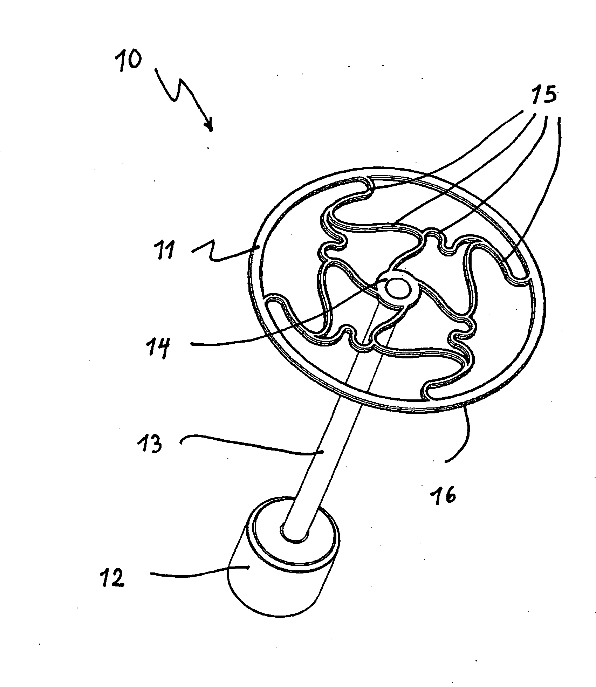

Die in den Figuren 1 und schematisch dargestellten **Gehörknöchelchenprothesen 10; 20** weisen jeweils am einen Ende ein **erstes Befestigungselement 11; 21** auf, welches in Form einer Kopfplatte zur Anlage am Trommelfell ausgebildet ist. Am anderen Ende der Gehörknöchelchenprothesen 10; 20 sitzt jeweils ein **zweites Befestigungselement 12; 22** zur mechanischen Verbindung der Prothese mit einem Glied oder Teilen eines Gliedes der Gehörknöchelchen-Kette oder mit dem Innenohr. Dazwischen ist ein die beiden Befestigungselemente 11,12 bzw. 21,22 Schall leitend miteinander verbindendes **Verbindungselement 13; 23** angeordnet, welches bei den gezeigten Ausführungs-formen in Form eines länglichen Schaftes ausgeführt ist.

Die Kopfplatten der Gehörknöchelchenprothesen 10; 20 bestehen bei den in der Zeichnung dargestellten Ausführungsformen aus einem **radial äußeren Ringbereich 16; 26,** einem **zentralen Ankoppelbereich 14; 24** zum mechanischen Ankoppeln der Kopfplatte an das Verbindungs-element 13; 23 sowie aus mehreren **Stegelementen 15; 25** zur radialen Verbindung des radial äußeren Ringbereichs 16; 26 mit dem zentralen Ankoppelbereich 14; 24. Erfindungsgemäß sind die Stegelemente 15; 25 geometrisch so gestaltet, dass sie bei lokaler Medialbewegung des Trommelfells dieser Medialbewegung lokal folgen, jedoch die Bewegung nicht an entfernte Bereiche der Kopfplatte weitergeben.

Insbesondere verlaufen bei den dargestellten Ausführungsformen die Stegelemente 15; 25 in der Kopfplattenebene nicht geradlinig, sondern auf mehrfach gekrümmten Kurven, und jedes Stegelement 15; 25 ist mit mindestens zwei anderen Stegelementen 15; 25 verbunden. Die Stegelemente 15; 25 weisen eine maximale Breite b und der radial äußere Ringbereich (16; 26) eine maximale Breite B auf, wobei 2b < B. Die Kopfplatten einschließlich Ringbereich 16; 26 weisen einen minimalen Durchmesser D auf, wobei b ≤ 0,05D, vorzugsweise b ≈ 0,03D.

Das zweite Befestigungselement 12 an dem der Kopfplatte entgegen gesetzten Ende der in Fig. 1 dargestellten Gehörknöchelchenprothese 10 ist im vorliegenden Ausführungsbeispiel als Kolben zur direkten Ankopplung der Gehörknöchelchenprothese 10 an das Innenohr ausgebildet.

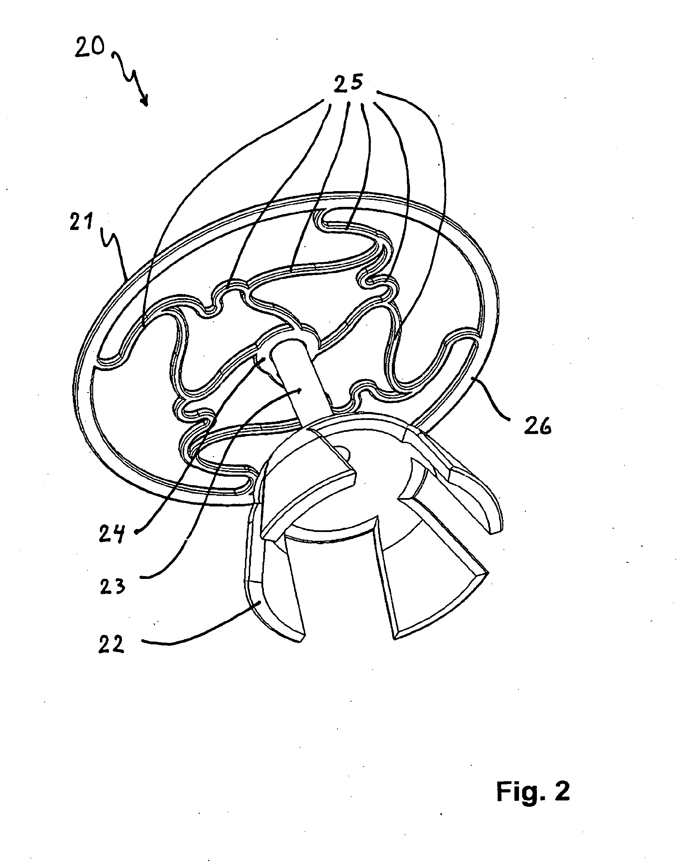

Bei der Ausführungsform nach Fig. 2 hingegen weist das zweite Befestigungselement 22 eine glockenartige Gestaltung auf und dient zur Befestigung der Gehörknöchelchenprothese 20 an einem Glied der Gehörknöchelchenkette, beispielsweise am Amboss oder am Steigbügel.

Die Massenverteilung der einzelnen Teile der erfindungsgemäßen Gehörknöchelchenprothese 10; 20 kann in Abhängigkeit von einem gewünschten, vorgebbaren Frequenzgang der Schallleitung im Mittelohr berechnet sein, um ein individuelles Tuning der SchallleitungsEigenschaften zu ermöglichen.

Bei in der Zeichnung nicht eigens dargestellten weiteren Ausführungsformen der erfindungsgemäßen Gehörknöchelchenprothese können die Stegelemente und/oder die äußeren Ringbereiche auch andere Geometrien besitzen, um die gewünschte Flexibilität der jeweiligen Kopfplatte zu erzielen. So können etwa zumindest einige Stegelemente eine Unterbrechung zwischen dem zentralen Ankoppelbereich und dem radial äußeren Ringbereich aufweisen. Auch kann der radial äußere Ringbereich einfach oder mehrfach unterbrochen gestaltet sein. Der radial äußere Ringbereich kann eine Schlangenlinienförmige Außenkontur und/oder eine einseitige Einbuchtung zur Aufnahme des Hammergriffs aufweisen.

## Patentansprüche

1. Gehörknöchelchenprothese (10; 20), die mindestens ein Glied oder Teile eines Gliedes der Gehörknöchelchenkette ersetzt oder überbrückt, wobei die Gehörknöchelchenprothese (10; 20) an ihrem einen Ende ein als Kopfplatte zur mechanischen Verbindung mit dem Trommelfell ausgebildetes erstes Befestigungselement (11; 21) und an ihrem anderen Ende ein zweites Befestigungselement (12; 22) zur mechanischen Verbindung mit einem Glied oder Teilen eines Gliedes der Gehörknöchelchen-Kette oder mit dem Innenohr sowie ein die beiden Befestigungselemente (11,12; 21,22) Schall leitend miteinander verbindendes Verbindungselement (13; 23) umfasst, und wobei die Kopfplatte einen radial äußeren Ringbereich (16; 26), einen radial inneren, insbesondere zentralen Ankoppelbereich (14; 24) zum mechanischen Ankoppeln der Kopfplatte an das Verbindungselement (13; 23) sowie mehrere Stegelemente (15; 25) zur radialen Verbindung des radial äußeren Ringbereichs (16; 26) mit dem radial inneren Ankoppelbereich (14; 24) aufweist,
**dadurch gekennzeichnet,**
**dass** die Kopfplatte eine Dicke t zwischen 0,01mm und 0,25mm sowie einen minimalen Durchmesser D zwischen 2mm und 5mm und die Stegelemente (15; 25) eine maximale Breite b zwischen 0,01mm und 0,2mm aufweisen, so dass die Stegelemente (15; 25) bei lokaler Medialbewegung des Trommelfells dieser Medialbewegung lokal folgen, jedoch die Bewegung nicht an entfernte Bereiche der Kopfplatte weitergeben.

2. Gehörknöchelchenprothese nach Anspruch 1, **dadurch gekennzeichnet, dass** die Stegelemente (15; 25) eine maximale Breite b und der radial äußere Ringbereich (16; 26) eine maximale Breite B aufweisen, und dass gilt: 2b < B.

3. Gehörknöchelchenprothese nach Anspruch 1 oder 2, **dadurch gekennzeichnet, dass** die Stegelemente (15; 25) eine maximale Breite b und die Kopfplatte einschließlich Ringbereich (16; 26) einen minimalen Durchmesser D aufweisen, und dass gilt: b ≤ 0,05D, vorzugsweise b ≈ 0,03D.

4. Gehörknöchelchenprothese nach einem der vorhergehenden Ansprüche, **dadurch gekennzeichnet, dass** die Stegelemente (15; 25) in der Kopfplattenebene nicht geradlinig, sondern auf mehrfach gekrümmten Kurven verlaufen.

5. Gehörknöchelchenprothese nach einem der vorhergehenden Ansprüche, **dadurch gekennzeichnet, dass** jedes Stegelement (15; 25) mit mindestens zwei anderen Stegelementen (15; 25) verbunden ist.

6. Gehörknöchelchenprothese nach einem der Ansprüche 1 bis 5, **dadurch gekennzeichnet, dass** die Stegelemente (15; 25) zwischen dem radial inneren Ankoppelbereich (14; 24) und dem radial äußeren Ringbereich (16; 26) ununterbrochen verlaufen.

7. Gehörknöchelchenprothese nach einem der Ansprüche 1 bis 5, **dadurch gekennzeichnet, dass** zumindest einige Stegelemente eine Unterbrechung zwischen dem radial inneren Ankoppelbereich und dem radial äußeren Ringbereich aufweisen.

8. Gehörknöchelchenprothese nach einem der Ansprüche 1 bis 7, **dadurch gekennzeichnet, dass** der radial äußere Ringbereich (16; 26) ununterbrochen in sich selbst geschlossen verläuft.

9. Gehörknöchelchenprothese nach einem der Ansprüche 1 bis 7, **dadurch gekennzeichnet, dass** der radial äußere Ringbereich mindestens eine, vorzugsweise mehrere Unterbrechungen aufweist.

10. Gehörknöchelchenprothese nach einem der Ansprüche 1 bis 9, **dadurch gekennzeichnet, dass** der radial äußere Ringbereich (16; 26) eine ovale oder kreisrunde Form aufweist.

11. Gehörknöchelchenprothese nach einem der Ansprüche 1 bis 9, **dadurch gekennzeichnet, dass** der radial äußere Ringbereich eine Schlangenlinienförmige Außenkontur aufweist.

12. Gehörknöchelchenprothese nach einem der Ansprüche 1 bis 9, **dadurch gekennzeichnet, dass** der radial äußere Ringbereich eine einseitige Einbuchtung zur Aufnahme des Hammergriffs aufweist.

13. Gehörknöchelchenprothese nach einem der vorhergehenden Ansprüche, **dadurch gekennzeichnet, dass** das Verbindungselement (13; 23) zwischen den Befestigungselementen (11, 12; 21, 22) als länglicher Schaft ausgeführt ist.

14. Gehörknöchelchenprothese nach Anspruch 13, **dadurch gekennzeichnet, dass** der längliche Schaft mindestens ein Gelenk, insbesondere ein Kugelgelenk, vorzugsweise eine Kugelgelenk-Kette aufweist.

15. Gehörknöchelchenprothese nach Anspruch 13, **dadurch gekennzeichnet, dass** der längliche Schaft einstückig durchgehend, insbesondere starr ausgeführt ist.

16. Gehörknöchelchenprothese nach einem der Ansprüche 1 bis 15, **dadurch gekennzeichnet, dass** das zweite Befestigungselement (22) als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

17. Gehörknöchelchenprothese nach Anspruch 16, **dadurch gekennzeichnet, dass** die Gehörknöchelchenprothese (20) einerseits am Trommelfell und andererseits am Amboss oder am Steigbügel befestigt ist.

18. Gehörknöchelchenprothese nach einem der Ansprüche 1 bis 15, **dadurch gekennzeichnet, dass** die Gehörknöchelchenprothese (10) mittels Perforation der Steigbügelfußplatte (=Stapedektomie bzw. Stapedotomie) und/oder mittels Eröffnung der menschlichen Hörschnecke (=Cochleotomie) an dem der Kopfplatte entgegen gesetzten anderen Ende direkt an das Innenohr angekoppelt ist, insbesondere über einen Kolben (12).

19. Gehörknöchelchenprothese nach einem der vorhergehenden Ansprüche, **dadurch gekennzeichnet, dass** die gesamte Gehörknöchelchenprothese (10; 20) oder Teile davon aus biokompatiblen Kunststoffen, insbesondere Silikon oder Polytetrafluorethylen (PTFE), und/oder aus FaserverbundWerkstoffen, insbesondere Kohlefasern hergestellt sind.

20. Gehörknöchelchenprothese nach einem der vorhergehenden Ansprüche, **dadurch gekennzeichnet, dass** Teile der Gehörknöchelchenprothese (10; 20) aus Titan und/oder aus Gold und/oder aus Tantal und/oder aus einer Legierung der genannten Metalle hergestellt sind.

21. Gehörknöchelchenprothese nach einem der vorhergehenden Ansprüche, **dadurch gekennzeichnet, dass** Teile der Gehörknöchelchenprothese (10; 20) aus einem Material mit Formgedächtnis (=memory effect), insbesondere aus Nitinol hergestellt sind.

22. Gehörknöchelchenprothese nach einem der vorhergehenden Ansprüche, **dadurch gekennzeichnet, dass** Teile der Gehörknöchelchenprothese (10; 20) aus einem Keramikmaterial hergestellt sind.

23. Gehörknöchelchenprothese nach einem der vorhergehenden Ansprüche, **dadurch gekennzeichnet, dass** zumindest abschnittsweise eine biologisch aktive Beschichtung, insbesondere eine wachstumshemmende und/oder eine wachstumsfördernde und /oder eine antibakteriell wirkende Beschichtung, vorgesehen ist.

24. Gehörknöchelchenprothese nach Anspruch 23, **dadurch gekennzeichnet, dass** die Kopfplatte eine wachstumsfördernde Beschichtung aufweist.

25. Gehörknöchelchenprothese nach Anspruch 18 sowie einem der Ansprüche 23 oder 24, **dadurch gekennzeichnet, dass** der Kolben (12) eine wachstumshemmende Beschichtung aufweist.

26. Gehörknöchelchenprothese nach einem der vorhergehenden Ansprüche, **dadurch gekennzeichnet, dass** die Massenverteilung der einzelnen Teile der Prothese in Abhängigkeit von einem gewünschten, vorgebbaren Frequenzgang der Schallleitung im Mittelohr berechnet ist.

27. Gehörknöchelchenprothese nach einem der vorhergehenden Ansprüche, **dadurch gekennzeichnet, dass** mindestens eine zusätzliche Masse in Abhängigkeit von einem gewünschten, vorgebbaren Frequenzgang der Schallleitung im Mittelohr an der Gehörknöchelchenprothese (10; 20) oder an einem Teil der Gehörknöchelchenkette befestigt ist, insbesondere mittels eines Clips.

28. Gehörknöchelchenprothese nach einem der vorhergehenden Ansprüche, **dadurch gekennzeichnet, dass** die Prothese mit einem aktiven Vibrationsteil eines aktiven, insbesondere implantierbaren Hörgeräts verbunden ist.

## Claims

1. Auditory ossicle prosthesis (10; 20) which replaces or bridges at least one member or part of a member of the ossicular chain, the auditory ossicle prosthesis (10; 20) including at its one end a first fastening element (11; 21) in the form of a head plate for mechanical connection to the tympanic membrane and at its other end a second fastening element (12; 22) for mechanical connection to a member or parts of a member of the ossicular chain, or to the inner ear, and a connecting element (13; 23) which connects the two fastening elements (11, 12; 21, 22) to one another in a sound conducting manner, and the head plate having a radially outer annular region (16; 26), a radially inner, in particular central, coupling region (14; 24) for mechanically coupling the head plate to the connecting element (13; 23), and a plurality of bridging elements (15; 25) for radially connecting the radially outer annular region (16; 26) to the radially inner coupling region (14; 24),
**characterised in that**
the head plate has a thickness t from 0.01 mm to 0.25 mm and a minimum diameter D from 2 mm to 5 mm, and the bridging elements (15; 25) have a maximum width b from 0.01 mm to 0.2 mm, so that, in the event of local medial movement of the tympanic membrane, the bridging elements (15; 25) follow this medial movement locally but do not transmit the movement to remote regions of the head plate.

2. Auditory ossicle prosthesis according to Claim 1, **characterised in that** the bridging elements (15; 25) have a maximum width b and the radially outer annular region (16; 26) has a maximum width B, and **in that** 2b < B.

3. Auditory ossicle prosthesis according to Claim 1 or 2, **characterised in that** the bridging elements (15; 25) have a maximum width b and the head plate including annular region (16; 26) has a minimum diameter D, and **in that** b ≤ 0.05D, preferably b ≈ 0.03D.

4. Auditory ossicle prosthesis according to any one of the preceding claims, **characterised in that** the bridging elements (15; 25) are not disposed rectilinearly but in multiply deflected curves in the plane of the head plate.

5. Auditory ossicle prosthesis according to any one of the preceding claims, **characterised in that** each bridging element (15; 25) is connected to at least two other bridging elements (15; 25).

6. Auditory ossicle prosthesis according to any one of Claims 1 to 5, **characterised in that** the bridging elements (15; 25) run uninterruptedly between the radially inner coupling region (14; 24) and the radially outer annular region (16; 26).

7. Auditory ossicle prosthesis according to any one of Claims 1 to 5, **characterised in that** at least some bridging elements have an interruption between the radially inner coupling region and the radially outer annular region.

8. Auditory ossicle prosthesis according to any one of Claims 1 to 7, **characterised in that** the radially outer annular region (16; 26) follows a path which is uninterrupted and closed in itself.

9. Auditory ossicle prosthesis according to any one of Claims 1 to 7, **characterised in that** the radially outer annular region has at least one interruption, preferably a plurality of interruptions.

10. Auditory ossicle prosthesis according to any one of Claims 1 to 9, **characterised in that** the radially outer annular region (16; 26) has an oval or circular shape.

11. Auditory ossicle prosthesis according to any one of Claims 1 to 9, **characterised in that** the radially outer annular region has a sinuous outer contour.

12. Auditory ossicle prosthesis according to any one of Claims 1 to 9, **characterised in that** the radially outer annular region has on one side an inwardly curved portion for receiving the malleus handle.

13. Auditory ossicle prosthesis according to any one of the preceding claims, **characterised in that** the connecting element (13; 23) between the fastening elements (11, 12; 21, 22) is configured as an elongated shaft.

14. Auditory ossicle prosthesis according to Claim 13, **characterised in that** the elongated shaft has at least one articulation, in particular a ball joint, preferably a chain of ball joints.

15. Auditory ossicle prosthesis according to Claim 13, **characterised in that** the elongated shaft has an integrally continuous, in particular rigid, configuration.

16. Auditory ossicle prosthesis according to any one of Claims 1 to 15, **characterised in that** the second fastening element (22) is in the form of a plate, a socket, a loop, a closed bell, a singly or multiply slotted bell or a clip for mechanical connection to a further member of the ossicular chain.

17. Auditory ossicle prosthesis according to Claim 16, **characterised in that** the auditory ossicle prosthesis (20) is fastened on one side to the tympanic membrane and on the other to the incus or to the stapes.

18. Auditory ossicle prosthesis according to any one of Claims 1 to 15, **characterised in that** the auditory ossicle prosthesis (10) is connected at the end opposite to the head plate directly to the inner ear, in particular via a piston (12), by means of perforation of the stapes footplate (= stapedectomy or stapedotomy) and/or by means of opening the human cochlea (= cochleotomy).

19. Auditory ossicle prosthesis according to any one of the preceding claims, **characterised in that** the whole auditory ossicle prosthesis (10; 20) or parts thereof are produced from biocompatible plastics materials, in particular silicone or polytetrafluoroethylene (PTFE), and/or from fibre-reinforced materials, in particular carbon fibres.

20. Auditory ossicle prosthesis according to any one of the preceding claims, **characterised in that** parts of the auditory ossicle prosthesis (10; 20) are produced from titanium and/or from gold and/or from tantalum and/or from an alloy of the aforementioned metals.

21. Auditory ossicle prosthesis according to any one of the preceding claims, **characterised in that** parts of the auditory ossicle prosthesis (10; 20) are produced from a material with shape memory effect, in particular from Nitinol.

22. Auditory ossicle prosthesis according to any one of the preceding claims, **characterised in that** parts of the auditory ossicle prosthesis (10; 20) are produced from a ceramic material.

23. Auditory ossicle prosthesis according to any one of the preceding claims, **characterised in that** a biologically active coating, in particular a growth-inhibiting and/or a growth-promoting coating and/or a coating with antibacterial effect, is provided at least zonally.

24. Auditory ossicle prosthesis according to Claim 23, **characterised in that** the head plate has a growth-promoting coating.

25. Auditory ossicle prosthesis according to Claim 18 and to either of Claims 23 and 24, **characterised in that** the piston (12) has a growth-inhibiting coating.

26. Auditory ossicle prosthesis according to any one of the preceding claims, **characterised in that** the mass distribution of the individual parts of the prosthesis is calculated as a function of a desired, predefinable frequency response of the sound conduction in the middle ear.

27. Auditory ossicle prosthesis according to any one of the preceding claims, **characterised in that** at least one additional mass is fastened, in particular by means of a clip, to the auditory ossicle prosthesis (10; 20) or to a part of the ossicular chain as a function of a desired, predefinable frequency response of the sound conduction in the middle ear.

28. Auditory ossicle prosthesis according to any one of the preceding claims, **characterised in that** the prosthesis is connected to an active vibration part of an active, in particular implantable, hearing aid.

## Revendications

1. Prothèse des osselets de l'oreille (10 ; 20), qui remplace ou qui meuble au moins un élément ou des parties d'un élément de la chaîne d'osselets de l'oreille, dans laquelle la prothèse des osselets de l'oreille (10 ; 20) comprend, à l'une de ses extrémités, un premier élément de fixation (11 ; 21) se présentant sous la forme d'une plaque de tête pour le raccordement mécanique avec la membrane tympanique et, à son autre extrémité, un second élément de fixation (12 ; 22) pour le raccordement mécanique avec un élément ou des parties d'un élément de la chaîne d'osselets de l'oreille ou avec l'oreille interne, ainsi qu'un élément de raccordement (13 ; 23) reliant les deux éléments de fixation (11, 12 ; 21, 22) l'un à l'autre de manière à conduire le son, et dans laquelle la plaque de tête présente une zone annulaire radialement externe (16 ; 26), une zone de couplage (14 ; 24) radialement interne, en particulier centrale, pour le couplage mécanique de la plaque de tête avec l'élément de raccordement (13 ; 23), ainsi que plusieurs traverses (15 ; 25) pour le raccordement radial de la zone annulaire radialement externe (16 ; 26) avec la zone de couplage radialement interne (14 ; 24), **caractérisée en ce que**
la plaque de tête présente une épaisseur t comprise entre 0,01 mm et 0,25 mm, ainsi qu'un diamètre minimal D compris entre 2 mm et 5 mm et les traverses (15 ; 25) présentent une largeur maximale b comprise entre 0,01 mm et 0,2 mm, de sorte que dans le cas d'un déplacement médial local de la membrane tympanique, les traverses (15 ; 25) suivent localement ce déplacement médial, mais ne transmettent pas le mouvement aux zones éloignées de la plaque de tête.

2. Prothèse des osselets de l'oreille selon la revendication 1, **caractérisée en ce que** les traverses (15 ; 25) présentent une largeur maximale b et la zone annulaire radialement externe (16 ; 26) présente une largeur maximale B et **en ce que** s'applique la relation 2b < B.

3. Prothèse des osselets de l'oreille selon la revendication 1 ou 2, **caractérisée en ce que** les traverses (15 ; 25) présentent une largeur maximale b et la plaque de tête, y compris la zone annulaire (16 ; 26), présente un diamètre minimal D, et **en ce que** s'applique la relation b ≤ 0,05 D, de préférence, b ≈ 0,03 D.

4. Prothèse des osselets de l'oreille selon l'une quelconque des revendications précédentes, **caractérisée en ce que** les traverses (15 ; 25) ne s'étendent pas de manière linéaire dans le plan de la plaque de tête, mais s'étendent selon des courbes incurvées plusieurs fois.

5. Prothèse des osselets de l'oreille selon l'une quelconque des revendications précédentes, **caractérisée en ce que** chaque traverse (15 ; 25) est raccordée à au moins deux autres traverses (15 ; 25).

6. Prothèse des osselets de l'oreille selon l'une quelconque des revendications 1 à 5, **caractérisée en ce que** les traverses (15 ; 25) s'étendent de manière ininterrompue entre la zone de couplage radialement interne (14 ; 24) et la zone annulaire radialement externe (16; 26).

7. Prothèse des osselets de l'oreille selon l'une quelconque des revendications 1 à 5, **caractérisée en ce qu'**au moins quelques traverses présentent une interruption entre la zone de couplage radialement interne et la zone annulaire radialement externe.

8. Prothèse des osselets de l'oreille selon l'une quelconque des revendications 1 à 7, **caractérisée en ce que** la zone annulaire radialement externe (16 ; 26) s'étend sans interruption de manière à être fermée sur elle-même.

9. Prothèse des osselets de l'oreille selon l'une quelconque des revendications 1 à 7, **caractérisée en ce que** la zone annulaire radialement externe présente au moins une interruption, de préférence, plusieurs interruptions.

10. Prothèse des osselets de l'oreille selon l'une quelconque des revendications 1 à 9, **caractérisée en ce que** la zone annulaire radialement externe (16 ; 26) présente une forme ovale ou circulaire.

11. Prothèse des osselets de l'oreille selon l'une quelconque des revendications 1 à 9, **caractérisée en ce que** la zone annulaire radialement externe présente un contour externe de forme sinueuse.

12. Prothèse des osselets de l'oreille selon l'une quelconque des revendications 1 à 9, **caractérisée en ce que** la zone annulaire radialement externe présente une indentation unilatérale pour recevoir le manche du marteau.

13. Prothèse des osselets de l'oreille selon l'une quelconque des revendications précédentes, **caractérisée en ce que** l'élément de raccordement (13 ; 23) se présente entre les deux éléments de fixation (11, 12 ; 21, 22) sous la forme d'une tige allongée.

14. Prothèse des osselets de l'oreille selon la revendication 13, **caractérisée en ce que** la tige allongée présente au moins une articulation, en particulier une articulation à rotule, de préférence, une chaîne d'articulations à rotule.

15. Prothèse des osselets de l'oreille selon la revendication 13, **caractérisée en ce que** la tige allongée est réalisée d'un seul tenant en continu, en particulier, sous forme rigide.

16. Prothèse des osselets de l'oreille selon l'une quelconque des revendications 1 à 15, **caractérisée en ce que** le second élément de fixation (22) se présente sous la forme d'une plaque, d'une douille, d'une boucle, d'une cloche fermée, d'une cloche à une ou à plusieurs fentes ou d'une agrafe, pour le raccordement mécanique avec un autre élément de la chaîne d'osselets de l'oreille.

17. Prothèse des osselets de l'oreille selon la revendication 16, **caractérisée en ce que** la prothèse des osselets de l'oreille (20) est fixée, d'une part, sur la membrane tympanique et, d'autre part, sur l'enclume ou l'étrier.

18. Prothèse des osselets de l'oreille selon l'une quelconque des revendications 1 à 15, **caractérisée en ce que** la prothèse des osselets de l'oreille (10) est couplée directement à l'oreille interne au moyen d'une perforation de la plaque de base de l'étrier (= stapédectomie ou stapédotomie) et/ou au moyen d'une incision de la cochlée humaine (= cochléotomie) à l'autre extrémité opposée à la plaque de tête, en particulier par le biais d'un piston (12).

19. Prothèse des osselets de l'oreille selon l'une quelconque des revendications précédentes, **caractérisée en ce que** toute la prothèse des osselets de l'oreille (10 ; 20) ou des parties de celle-ci sont fabriquées en matériaux synthétiques biocompatibles, en particulier, en silicone ou en poly(tétrafluorure d'éthylène) (PTFE) et/ou en matériaux de fibres composites, en particulier en fibres de carbone.

20. Prothèse des osselets de l'oreille selon l'une quelconque des revendications précédentes, **caractérisée en ce que** des parties de la prothèse des osselets de l'oreille (10 ; 20) sont fabriquées en titane et/ou en or et/ou en tantale et/ou en un alliage des métaux cités.

21. Prothèse des osselets de l'oreille selon l'une quelconque des revendications précédentes, **caractérisée en ce que** des parties de la prothèse des osselets de l'oreille (10 ; 20) sont fabriquées en un matériau à mémoire de forme (= memory effect), en particulier en nitinol.

22. Prothèse des osselets de l'oreille selon l'une quelconque des revendications précédentes, **caractérisée en ce que** des parties de la prothèse des osselets de l'oreille (10 ; 20) sont fabriquées en un matériau céramique.

23. Prothèse des osselets de l'oreille selon l'une quelconque des revendications précédentes, **caractérisée en ce qu'**il est prévu, du moins par sections, un revêtement biologiquement actif, en particulier un revêtement inhibant la croissance et/ou un revêtement favorisant la croissance et/ou un revêtement à action antibactérienne.

24. Prothèse des osselets de l'oreille selon la revendication 23, **caractérisée en ce que** la plaque de tête présente un revêtement favorisant la croissance.

25. Prothèse des osselets de l'oreille selon la revendication 18 et selon l'une quelconque des revendications 23 ou 24, **caractérisée en ce que** le piston (12) présente un revêtement inhibant la croissance.

26. Prothèse des osselets de l'oreille selon l'une quelconque des revendications précédentes, **caractérisée en ce que** la distribution de masse des parties individuelles de la prothèse est calculée en fonction d'une réponse harmonique prédéfinissable souhaitée de la conduction acoustique dans l'oreille moyenne.

27. Prothèse des osselets de l'oreille selon l'une quelconque des revendications précédentes, **caractérisée en ce qu'**au moins une masse supplémentaire est fixée, en fonction d'une réponse harmonique prédéfinissable souhaitée de la conduction acoustique dans l'oreille moyenne, sur la prothèse des osselets de l'oreille (10 ; 20) ou sur une partie de la chaîne d'osselets de l'oreille, en particulier, au moyen d'une agrafe.

28. Prothèse des osselets de l'oreille selon l'une quelconque des revendications précédentes, **caractérisée en ce que** la prothèse est raccordée à une partie vibratoire active d'un appareil auditif actif, en particulier implantable.
